# FASCICULE DE BREVET EUROPEEN

(11) **EP 3 860 333 B1**
(45) Date de publication et mention de la délivrance du brevet: **25.11.2026**
(21) Numéro de dépôt: 19778549.6
(22) Date de dépôt: 02.10.2019
(51) Int. Cl.: A01K 11/00, A61B 10/02

(54) **PINCE POUR LA MANIPULATION D'UN DISPOSITIF D'IDENTIFICATION D'UN ANIMAL ET/OU DE PRÉLÈVEMENT D'UN TISSU D'UN ANIMAL COMPRENANT DES MOYENS DE MAINTIEN À MOYENS D'ACTIONNEMENT DÉPORTÉS**
KLEMME ZUR HANDHABUNG EINER TIERIDENTIFIKATIONSVORRICHTUNG UND/ODER EINER TIERGEWEBEENTNAHMEVORRICHTUNG MIT HALTEMITTELN MIT FERNBETÄTIGUNGSMITTELN
CLAMP FOR HANDLING AN ANIMAL IDENTIFICATION DEVICE AND/OR AN ANIMAL TISSUE SAMPLING DEVICE COMPRISING HOLDING MEANS WITH REMOTE ACTUATING MEANS

(30) Priorité: 03.10.2018 FR 1859184
(43) Date de publication de la demande: 11.08.2021
(73) Titulaire: Allflex Europe SAS, 35500 Vitré (FR)
(72) Inventeur: TEYCHENE, Bruno, 81430 Mouzieys-Teulet (FR); BLANC-TAILLEUR, Philippe, 31400 Toulouse (FR)
(74) Mandataire: Intervet International B.V.
(86) Numéro de dépôt international: PCT/EP2019/076775
(87) Numéro de publication internationale: WO 2020/070227

(56) Documents cités:
- WO-A1-01/72117
- WO-A1-2009/127541
- WO-A1-2012/013429
- GB-A- 2 044 684
- US-A- 4 030 506

## Description

### 1. Domaine de l'invention

Le domaine de l'invention est celui de la conception et de la fabrication de pinces permettant de fixer, à l'oreille d'un animal, un dispositif d'identification portant des informations d'identifications visuelles et/ou électroniques relative à cet animal. Elle concerne également le domaine des pinces permettant de manipuler un dispositif de prélèvement d'un tissu d'un animal.

Plus précisément, l'invention concerne la manière de maintenir solidaires des mâchoires de la pince les éléments qui composent un tel dispositif d'identification et/ou de prélèvement d'un tissu.

### 2. Art antérieur

Afin d'assurer l'identification animale notamment pour répondre aux exigences sanitaire et de traçabilité, des dispositifs d'identification sont posés sur les animaux d'élevages notamment porcins, bovins, ovins/caprins ou autres. L'art antérieur est représenté par WO01/72117 A1, GB 2 044 684 A, WO 2012/013429 A1 et US 4 030 506 A qui divulguent une pince pour la manipulation d'un dispositif d'identification d'un animal et/ou de prélèvement d'un tissu d'un animal.

Au rang de ces dispositifs d'identification figurent les boucles d'oreille.

Ces boucles d'oreille comprennent classiquement un élément femelle et un élément mâle destinés à être placés de part et d'autre d'une oreille et à coopérer entre eux pour maintenir la boucle solidaire de l'oreille.

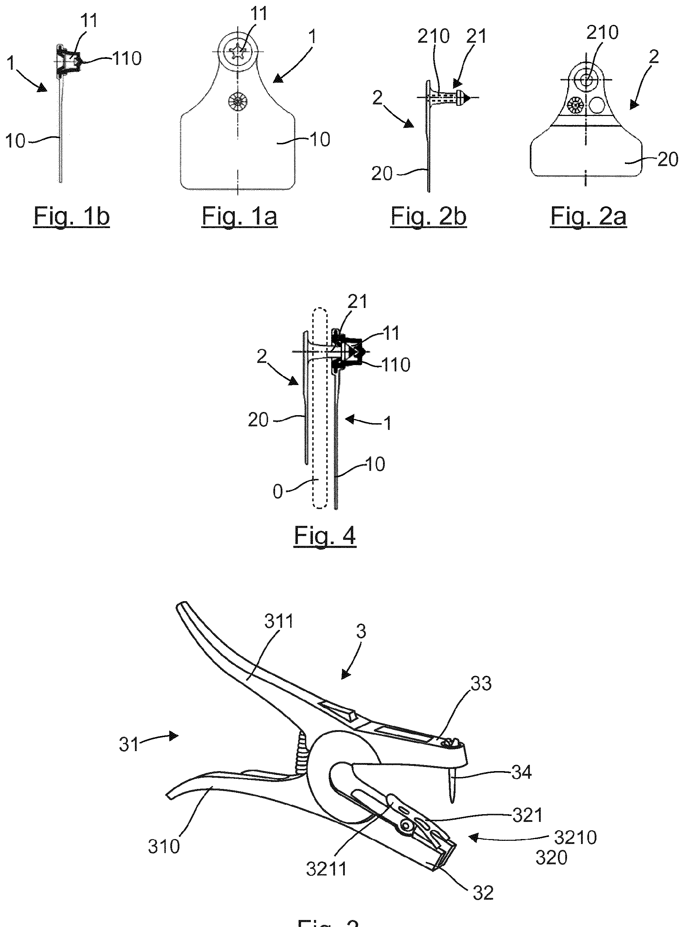

Ainsi que cela est représenté aux figures 1a et 1b, illustrant respectivement une vue de face et une vue de côté d'un exemple d'élément femelle, un tel élément 1 comprend ici une plaquette support d'informations 10 prolongée à l'une de ses extrémités par un logement de verrouillage 11 refermé par un chapeau 110.

Ainsi que cela est représenté aux figures 2a et 2b, illustrant respectivement une vue de face et une vue de côté d'un exemple d'élément mâle, un tel élément 2 comprend ici une plaquette 20 prolongée à l'une de ses extrémités par une pointe de verrouillage 21 prévue pour coopérer avec le logement de verrouillage 11 de l'élément femelle 1.

Au moins un, sinon les deux, éléments femelle ou mâle peuvent porter des informations permettant notamment l'identification de l'animal. Ces informations peuvent par exemple consister en des inscriptions lisibles visuellement (inscriptions alpha numériques, code à barres...) et/ou en des informations enregistrées sur un support électronique comme par exemple une étiquette RFID ou autre.

La mise en place d'une telle boucle d'oreille suppose l'utilisation d'une pince conçue à cet effet telle que celle illustrée à la figure 3.

Une pince 3 de ce type comprend une poignée 31 comprenant deux branches 310, 311 articulées entre elles et prolongée chacune respectivement par une mâchoire 33, 32.

La mâchoire 33 porte un pointeau 34 le long duquel un élément mâle 2 peut être emmanché à force en insérant le pointeau 34 dans un logement de forme complémentaire 210 ménagé à l'intérieur de la pointe de verrouillage 21.

La mâchoire 32 comprend un réceptacle 320 apte à loger le chapeau 110 d'un élément femelle 1. Elle porte également une bride 321 (encore appelée pédale) destinée à maintenir un élément femelle 1 solidaire de la mâchoire 32.

Cette bride 321 comprend une extrémité en forme de fourche 3210 avec une ouverture frontale permettant le passage de la pointe de verrouillage 21 d'un élément mâle 2. Elle se prolonge vers la zone d'articulation des branches 310, 311 par une languette d'actionnement 3211 sur laquelle un opérateur peut agir pour éloigner ou rapprocher la fourche 3210 de l'extrémité de la mâchoire 32, un ressort tendant à rapprocher la fourche 3210 de l'extrémité de la mâchoire 32.

La mise en place d'une telle boucle d'identification sur l'oreille d'un animal est obtenue de la manière suivante.

Un opérateur saisit d'une première de ses mains la poignée 31 puis emmanche avec sa deuxième main un élément mâle 2 le long du pointeau 34. Il saisit ensuite dans sa deuxième main un élément femelle 1 et le rapproche de la mâchoire 32 en vue d'insérer le chapeau 110 dans le réceptacle 320. Pour cela, il déplace sa première main de sorte à saisir la pince non plus par la poignée 31 mais au niveau de la mâchoire 32 de sorte à pouvoir agir sur la languette d'actionnement 3211 pour soulever de sa première main la fourche 3210 et ainsi dégager l'accès au réceptacle 320. L'opérateur peut alors positionner de sa deuxième main l'élément femelle 1 entre la mâchoire 32 et la bride 321 en insérant le chapeau 110 dans le logement 320. L'opérateur relâche enfin la languette d'actionnement 3211 si bien que la bride 321 se rapproche de la mâchoire 32 sous l'effet du ressort pour maintenir l'élément femelle 1 solidaire de la mâchoire 32.

L'opérateur place ensuite une oreille O entre les deux mâchoires 32, 33 puis actionne la poignée 31 pour rapprocher les mâchoires l'une de l'autre jusqu'à ce que la pointe de verrouillage 21 vienne se loger dans le logement de verrouillage 11.

L'élément femelle 1 et l'élément mâle 2 enserrent ainsi l'oreille O si bien que la boucle y est maintenue de manière irréversible, comme cela est représenté à la figure 4.

Cette technique, bien qu'efficace et relativement simple à mettre en œuvre, peut néanmoins encore être améliorée.

En effet, il n'est pas toujours évident pour l'opérateur de maintenir la pince et de manipuler la bride 321 d'une main et de manipuler l'élément femelle 1 de l'autre main. Pour cela, l'opérateur saisit généralement d'une main la pince non pas par la poignée 31 mais dans la région de l'articulation des deux branches 310, 311 de sorte à pouvoir tenir la pince et manipuler d'une même main la languette d'actionnement 3211 de la bride 321 et manipuler de l'autre main l'élément femelle 1. Cette pratique n'est toutefois pas optimale dans la mesure où l'opérateur ne tient alors pas confortablement la pince par sa poignée 31, ce qui exerce un impact négatif sur la productivité. Il existe donc un besoin d'améliorer notamment l'ergonomie des pinces permettant la mise en place de boucles d'oreille d'identification. Ce besoin existe aussi pour les pinces permettant de manipuler un dispositif de prélèvement d'un tissu d'un animal, un tel dispositif comprenant un tube de prélèvement (récipient) devant être maintenu de manière réversible à l'une des mâchoires de la pince au cours du prélèvement.

### 3. Objectifs de l'invention

L'invention a notamment pour objectif d'apporter une solution efficace à au moins certains de ces différents problèmes.

En particulier, selon au moins un mode de réalisation, un objectif de l'invention est de fournir une pince pour la manipulation d'un dispositif d'identification d'un animal et/ou de prélèvement d'un tissu d'un animal qui permet de faciliter le travail d'un opérateur œuvrant à cette tâche, et notamment d'améliorer l'ergonomie.

Notamment, l'invention a pour objectif, selon au moins un mode de réalisation, de fournir une telle pince qui permet à l'opérateur de maintenir confortablement la pince par sa poignée tout au long de la mise en place d'un boucle d'identification sur l'oreille d'un animal, et en particulier lors de la mise en place des éléments mâle et/ou femelle sur la pince, ou de la réalisation d'un prélèvement de tissus.

Un autre objectif de l'invention est, selon au moins un mode de réalisation, de fournir une telle pine qui soit simple de conception et/ou robuste et/ou économique et/ou dont l'utilisation concourt à améliorer la productivité.

### 4. Présentation de l'invention

Pour ceci, l'invention propose une pince selon la revendication 1.

Ainsi, selon cet aspect de l'invention, un opérateur peut d'une même main enserrer confortablement la poignée et agir sur les moyens d'actionnement pour obtenir le maintien d'un des éléments d'une boucle d'identification. Il peut ainsi de l'autre de ses mains mettre en place l'élément de la boucle d'identification à maintenir dans la pince. Dans le cas d'un dispositif de prélèvement, l'opérateur peut ainsi manipuler confortablement la pince d'une main alors qu'il approche de l'autre le tube de prélèvement pour le maintenir solidaire de la pince.

On améliore ainsi l'ergonomie, en permettant à l'opérateur de manipuler la pince confortablement d'une main et de manipuler les parties de la boucle ou du tube de prélèvement de l'autre main.

La technique selon l'invention permet ainsi d'améliorer le confort de l'opérateur et d'améliorer la productivité en lui faisant gagner du temps.

Selon une variante possible, ledit dispositif d'identification comprend un élément femelle et un élément mâle destinés à être placés de part et d'autre d'une oreille d'un animal et à être rapproché pour coopérer entre eux et maintenir ledit dispositif d'identification solidaire de ladite oreille, lesdits éléments femelle et mâle étant chacun destiné à être supportés par une desdites mâchoires, lesdits moyens de maintien étant mobiles entre :
- une position de libération dans laquelle lesdits moyens de maintien sont éloignés de ladite mâchoire correspondante, et
- une position de maintien dans laquelle lesdits moyens de maintien sont rapprochés de ladite mâchoire correspondante pour maintenir le cas échéant ledit élément mâle ou ledit élément femelle entre lesdits moyens de maintien et ladite mâchoire correspondante.

Dans ce cas, un élément d'un dispositif d'identification peut être efficacement maintenu entre les moyens de maintien et la mâchoire correspondante.

Selon une variante possible, lesdits moyens de maintien sont montés mobiles autour d'un premier axe de rotation entre leur position de maintien et leur position de libération.

Selon une variante possible, lesdits moyens de maintien sont montés mobiles selon un axe de translation entre leur position de maintien et leur position de libération.

Selon une variante possible, ladite poignée comprend deux branches montées mobile en rotation selon un deuxième axe de rotation, ledit premier axe de rotation (Z) étant essentiellement parallèle audit deuxième axe de rotation ou ledit axe de translation (B) étant essentiellement orthogonal audit deuxième axe de rotation.

Dans des variantes, l'axe de rotation des moyens de maintien pourra être confondu avec celui des branches ou l'axe de translation des moyens de maintien pourra être sécant avec l'axe de rotation des branches. L'axe de rotation des moyens de maintien pourra être confondu avec celui des branches ou l'axe de translation des moyens de maintien pourra également être distant de l'axe de rotation des branches.

Une pince selon l'invention peut comprendre des moyens de rappel élastique tendant à ramener lesdits moyens de maintien dans une de leur position, les moyens d'actionnement permettant de les déplacer dans l'autre de leur position.

Selon une variante possible, lesdits moyens de maintien comprennent une bride mobile entre :
- ladite position de libération dans laquelle ladite bride et ladite mâchoire correspondante sont éloignées pour libérer ledit élément mâle ou ledit élément femelle, et
- une position de maintien dans laquelle ladite bride et ladite mâchoire correspondante sont rapprochées pour enserrer ledit élément mâle ou ledit élément femelle entre ladite bride et ladite mâchoire correspondante.

Selon une variante possible, lesdits moyens d'actionnement comprennent au moins un levier s'étendant à l'extrémité de ladite poignée située du côté desdites mâchoires de manière à pouvoir être actionné par un doigt d'une main empoignant ladite poignée.

Ceci permet d'améliorer l'ergonomie.

Selon une variante possible, ledit au moins un levier est solidaire d'un flanc dont est solidaire ladite bride.

Selon une variante possible, ledit levier forme avec ledit flanc et ladite bride un ensemble monobloc.

Selon une variante possible, ledit flanc porte au moins deux lumières s'étirant le long d'un même cercle dont le centre passe par le premier axe de rotation des moyens de maintien, lesdites lumière logeant des axes de solidarisation dudit flanc au reste de ladite pince.

Selon une variante possible, ledit flanc porte au moins deux lumières oblongue s'étirant le long dudit axe de translation des moyens de maintien, lesdites lumières oblongues logeant des axes de solidarisation dudit flanc au reste de ladite pince.

Selon une variante possible, lesdites lumières ou lesdites lumières oblongues sont latéralement décalées dudit premier axe de rotation en direction de ladite poignée.

Ceci permet de dégager plus d'espace entre les mâchoires pour y accueillir une oreille.

Selon une variante possible, ledit dispositif de prélèvement comprend un récipient apte à recevoir un prélèvement, lesdits moyens de maintien comprenant deux mors placés en regard l'un de l'autre montés mobiles entre :
- une position de libération dans laquelle lesdits mors sont éloignés pour libérer ledit récipient, et
- une position de maintien dans laquelle lesdits mors sont rapprochés pour maintenir ledit récipient solidaire de ladite mâchoire correspondante.

Selon l'invention, les moyens de maintien comprennent au moins un élément de maintien (par exemple une bride ou une paire de mores) mobile entre :
- une position de libération dans laquelle il ne maintient pas solidaire de ladite mâchoire ledit élément dudit dispositif d'identification et/ou de prélèvement, et
- une position de maintien dans laquelle il maintient par enserrement solidaire de ladite mâchoire ledit élément dudit dispositif d'identification et/ou de prélèvement.

Selon l'invention, ladite pince comprend des moyens d'actionnement pour déplacer ledit au moins un élément de maintien d'une à l'autre de ses positions, lesdits moyens d'actionnement comprenant au moins un élément d'actionnement (par exemple un levier ou un bouton) actionnable manuellement depuis la poignée au moyen d'une main enserrant la poignée.

### 5. Liste des figures

D'autres caractéristiques et avantages de l'invention apparaîtront à la lecture de la description suivante de modes de réalisation particuliers, donnés à titre de simples exemples illustratifs et non limitatifs, et des dessins annexés parmi lesquels :
- les figures 1a et 1b illustrent des vues de face et de côté d'un élément femelle d'une boucle d'identification ;
- les figures 2a et 2b illustrent des vues de face et de côté d'un élément mâle d'une boucle d'identification ;
- la figure 3 illustre une pince selon l'art antérieur pour la mise en place d'une boucle d'identification ;
- la figure 4 illustre la solidarisation d'une boucle d'identification à l'oreille d'un animal ;
- la figure 5 illustre une vue de côté d'une pince selon l'invention, en position fermée, sur laquelle une boucle d'identification est mise en place, et dont les moyens de maintien sont en position de maintien ;
- la figure 6 illustre une vue partielle en couple longitudinale de la pince de la figure 5 ;
- les figures 7 et 8 illustrent des vues en perspective d'une bride selon l'invention ;
- la figue 9 illustre une vue de côté d'une pince selon l'invention en position fermée, sur laquelle une boucle d'identification est mise en place, avec la bride en position de libération ;
- la figure 10 illustre une vue de côté d'une pince selon l'invention en position ouverte, sur laquelle une boucle d'identification est mise en place, avec la bride en position de libération ;
- la figure 11 illustre une vue de côté d'une pince selon l'invention en position ouverte, sur laquelle une boucle d'identification est mise en place, avec la bride en position de maintien ;
- la figure 12 illustre une vue de détail des figures 10 et 11 ;
- les figures 13 et 14 illustrent des vues de côté d'une pince en position fermée à moyens de maintien déplaçables en translation respectivement en position de maintien et en position de libération ;
- la figure 15 illustre un détail des lumières oblongues de la pince illustrée aux figures 13 et 14 ;
- les figures 16 et 17 illustrent une variante d'une pince pour la manipulation d'un dispositif de prélèvement de tissu.

### 6. Description de modes de réalisation particuliers

### 6.1. Architecture d'une pince

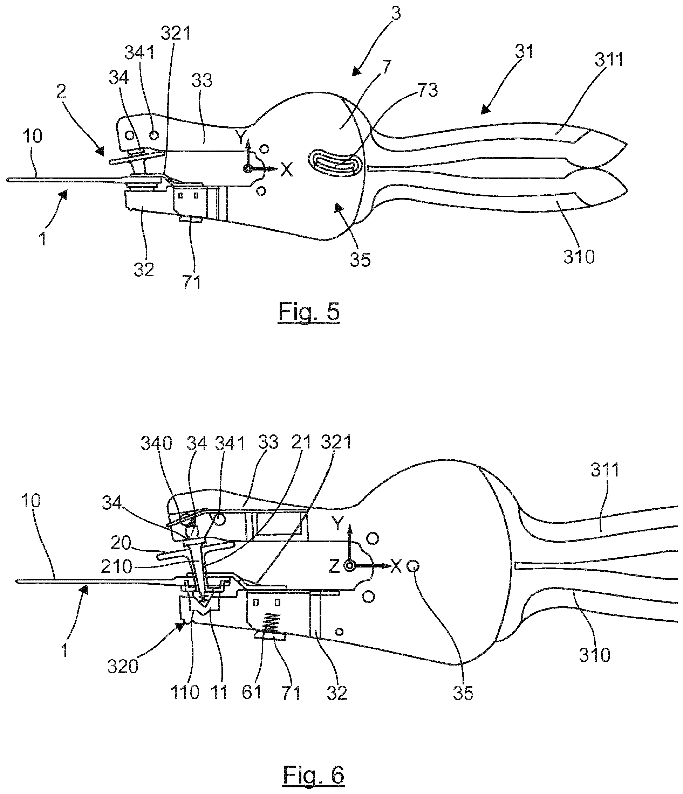

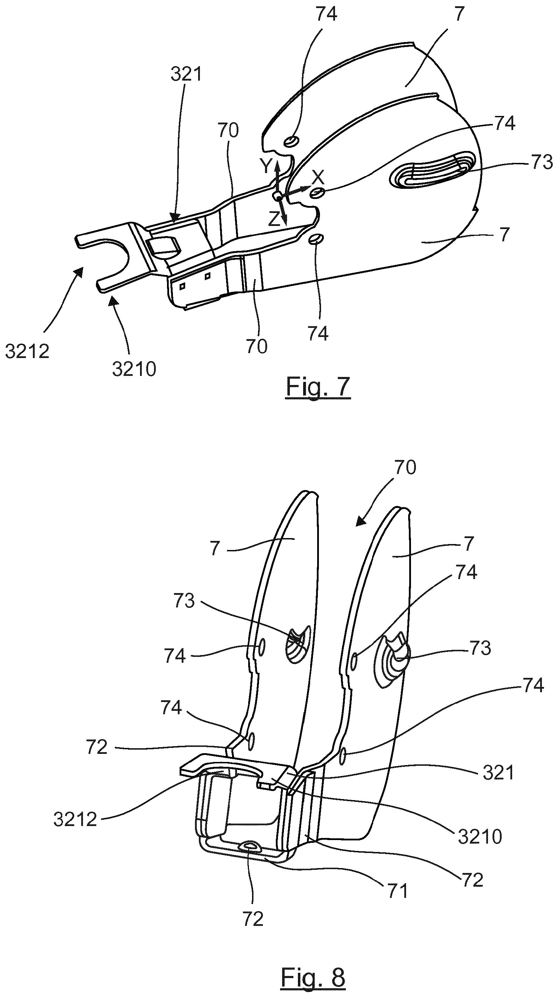

On présente en relation avec les figures 5 à 12, un mode de réalisation d'une pince selon l'invention pour la mise en place d'une boucle d'identification sur l'oreille d'un animal.

Une telle pince 3 comprend classiquement une poignée 31 comprenant deux branches 310, 311 prolongées chacune par une mâchoire 32, 33.

Les deux branches 310, 311 sont classiquement reliées par une articulation 35 comprenant une liaison pivot de manière telle qu'un rapprochement des branches 310, 311 en les déplaçant l'une par rapport à l'autre selon l'axe de cette liaison pivot s'accompagne d'un rapprochement des mâchoires 32, 33 l'une de l'autre, et inversement.

La mâchoire 33 porte un pointeau 34 le long duquel un élément mâle 2 peut être emmanché à force en insérant le pointeau 34 dans un logement de forme complémentaire 210 ménagé à l'intérieur de la pointe de verrouillage 21.

Dans ce mode de réalisation, le pointeau 34 est monté sur un support 340 monté mobile dans la mâchoire 33 selon l'axe 341 d'une liaison pivot essentiellement parallèle à l'axe de l'articulation 35 des branches 310, 311. Dans une variante, le pivot 34 pourra être fixe par rapport à la mâchoire 33 dont il est solidaire.

La mâchoire 32 comprend un réceptacle 320 apte à loger le chapeau 110 d'un élément femelle 1. Elle porte également une bride 321 (encore appelée pédale) destinée à maintenir un élément femelle 1 solidaire de la mâchoire 32.

Cette bride 321 comprend une extrémité en forme de fourche 3210 avec une ouverture frontale 3212.

La bride 320 est solidaire, à son extrémité opposée à celle de la fourche 3210, de deux flancs 7 essentiellement parallèles et distants l'un de l'autre pour manéger un espace intérieur 70.

Ces deux flancs 7 se prolongent chacun par une extension 72 à l'extrémité de laquelle ils sont reliés par un pont 71. Un bossage 72 est ménagé sur le pont 71.

Un levier d'actionnement 73 est ménagé à la surface de chaque flanc 7. Il s'agit ici plus particulièrement d'un bossage d'actionnement. Dans une variante, un seul flanc pourra présenter un levier d'actionnement. La fait de mettre en œuvre un bossage d'actionnement 73 sur chaque flanc permet un actionnement tant par un droitier que par un gaucher ou l'actionnement par deux doigts différents (par exemple le pouce et l'index). Dans une autre variante, un seul flanc pourra être mis en œuvre. La surface des bossages d'actionnement 73 destinée à être au contact d'un doigt peut être incurvée pour épouser sensiblement la forme du doigt et ainsi améliorer l'ergonomie.

L'espace intérieur 70 est prévu pour loger le reste de la pince 1 au niveau de l'articulation 35 des branches 310, 311.

Les deux flancs 7, les extensions 72 et le pont 71 sont formés d'une tôle emboutie et pliée à laquelle la bride 320 est solidarisée par sertissage à l'extrémité des extensions 72 pour former un ensemble monobloc. Un autre moyen de fixation que le sertissage, tel que le soudage, le vissage, le collage ou autre pourra être mis en œuvre pour assurer la solidarisation de la bride. Dans une variante, la bride pourra également être formés dans la tôle des flancs et être pliée.

Chaque flanc 7 est traversé par deux lumières 74 (il est toutefois possible d'en mettre en œuvre une seule). Ces lumières 74 sont oblongues et s'étendent le long d'un cercle C de centre A. Elles présentent essentiellement la forme d'une portion d'anneau (portion de lune) Elles logent des axes 75 de solidarisation des flancs 7 au reste de la pince 1.

La bride 320 est ainsi montée mobile en rotation autour d'un premier axe de rotation Z passant par le centre A du cercle C et essentiellement parallèle à l'axe de la liaison pivot 35 de l'articulation des branches 310, 311.

Dans ce cas, les axes de rotation de la bride 320 et des branches 310, 311 sont différents. Ils pourraient toutefois être confondus.

Les lumières oblongues 74 sont latéralement décalées du premier axe de rotation Z en direction de la poignée 31, ce qui permet de dégager plus d'espace entre les mâchoires 32, 33 pour y accueillir une oreille.

La bride 321 est ainsi mobile entre :
- une position de libération dans laquelle la bride 320 et la mâchoire 32 sont éloignées pour libérer l'élément femelle placé entre elles, et
- une position de maintien dans laquelle la bride 320 et la mâchoire 32 sont rapprochées pour maintenir l'élément femelle enserré entre la bride et la mâchoire 32.

Un ressort de compression 61 placé sur le bossage 73 entre le pont 71 et la mâchoire 32 tend à ramener la bride 320 dans sa position de maintien.

Un opérateur peut agir sur les bossages d'actionnement 73 pour modifier la position de la bride 320.

Les bossages d'actionnement 73 sont placés de telle sorte que lorsqu'un opérateur enserre d'une main la poignée 31, ces bossages d'actionnement « tombent » sous un doigt de cette main, notamment le pouce ou l'index. Les bossages d'actionnement 73 peuvent ainsi être actionnés par un doigt de la main enserrant la poignée 31.

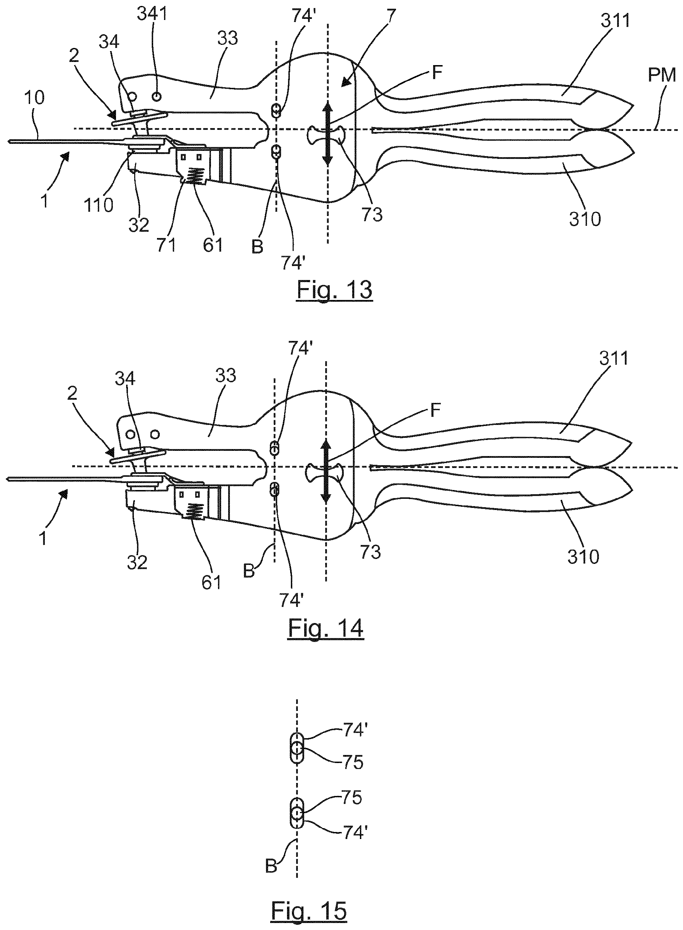

Dans une variante, illustrée aux figures 13 à 15, la bride 320 est montée mobile entre ses positions selon un axe de translation B essentiellement orthogonal à un plan médian PM de la pince s'étendant entre ses mâchoires et ses branches lorsque la pince est fermée et passant par l'axe de rotation de l'articulation 35 des branches 32, 33.

Dans ce cas, les lumières oblongues 74' s'étirent selon cet axe de translation B.

Bien entendu, l'axe de translation pourra être orienté différemment (il pourra par exemple être incliné par rapport au plan médian PM, voir s'étendre dans ce plan - dans ce cas le déplacement de la bride de l'une à l'autre de ses positions peut se faire par un coulissement de droite à gauche sur les figures-, et orthogonal à l'axe de l'articulation des branches) dès lors qu'il permettra de réaliser un déplacement de la bride de l'une à l'autre de ses positions. Dans le cas d'un coulissement dans le plan PM ou dans un plan essentiellement parallèle à celui-ci, indiquer que dans la position de relâchement, les moyens de maintien se trouvent éloignés de la mâchoire correspondante, et que dans la position de maintien, ils se trouvent rapprochés de la mâchoire correspondante, signifie qu'ils sont respectivement rapprochés ou éloignés de l'extrémité de la mâchoire.

### 6.2. Fonctionnement d'une pince

La mise en place d'une boucle d'identification sur l'oreille d'un animal est obtenue de la manière suivante.

Un opérateur saisit d'une première de ses mains la poignée 31 puis emmanche avec sa deuxième main un élément mâle 2 le long du pointeau 34.

La bride 321 se trouve dans sa position de maintien dans laquelle elle est maintenue sous l'effet du ressort 61. La bride 320 se trouve alors rapprochée de la mâchoire 32.

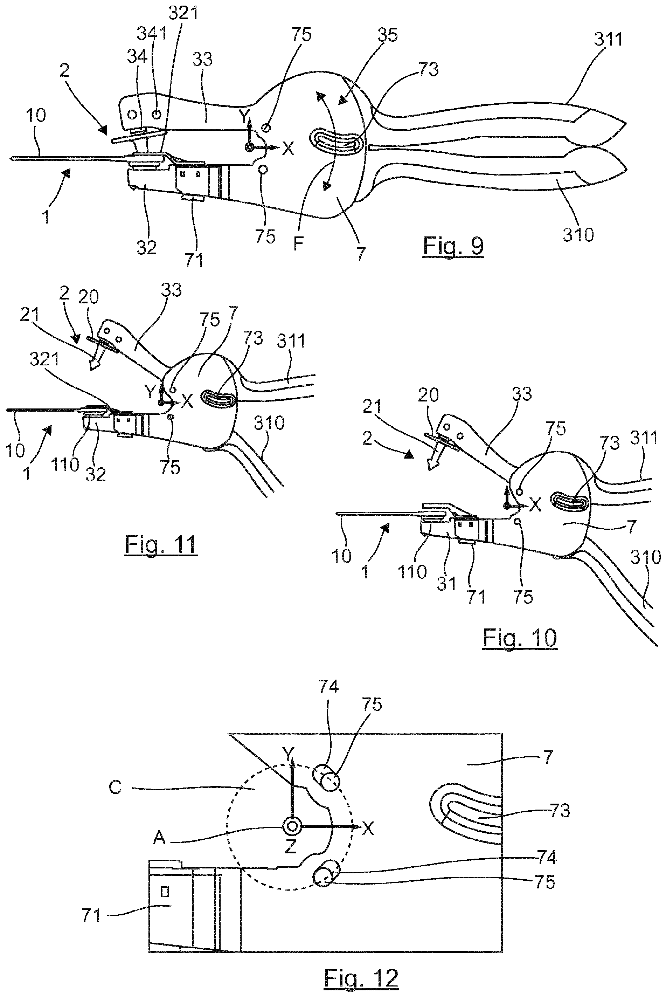

L'opérateur agit ensuite sur un bossage d'actionnement 73 d'un doigt de la main avec laquelle il enserre confortablement la poignée 31 de manière à placer la bride 321 dans sa position de libération dans laquelle elle est éloignée de la mâchoire 32 par un mouvement de rotation ou de translation (cf. flèches F aux figures 9 et 13). L'accès au logement 320 est alors libéré.

L'opérateur saisit ensuite dans sa deuxième main un élément femelle 1, le rapproche de la mâchoire 32 puis positionne l'élément femelle 1 entre la mâchoire 32 et la bride 321 en insérant de cette main le chapeau 110 dans le logement 320.

L'opérateur relâche le bossage d'actionnement 73 si bien que la bride 321 retourne dans sa position de maintien sous l'effet du ressort 61. L'élément femelle 1 se trouvent enserré entre la bride et la mâchoire 32 et est ainsi maintenu en position.

L'opérateur place ensuite une oreille O entre les deux mâchoires 32, 33 puis actionne la poignée 31 pour rapprocher les mâchoires l'une de l'autre jusqu'à ce que la pointe de verrouillage 21 vienne se loger dans le logement de verrouillage 11.

L'élément femelle 1 et l'élément mâle 2 enserrent ainsi l'oreille O si bien que la boucle y est maintenue de manière irréversible, comme cela est représenté à la figure 4.

L'opérateur relâche ensuite la poignée 31 pour rouvrir la pince puis la dégager de l'animal.

Un système d'ouverture automatique de la pince peut également être prévu. Un tel système permet, à la fin de la course de fermeture des branches 310, 311, d'obtenir une réouverture des mâchoires 32, 33 sans rouvrir les branches 310, 311.

### 6.3. Variantes

Dans le mode de réalisation décrit plus haut, les moyens de rappel tendent à ramener la bride dans sa position de maintien et les moyens d'actionnement sont actionnés pour les placer dans leur position de libération. Ce fonctionnement pourrait être inversé.

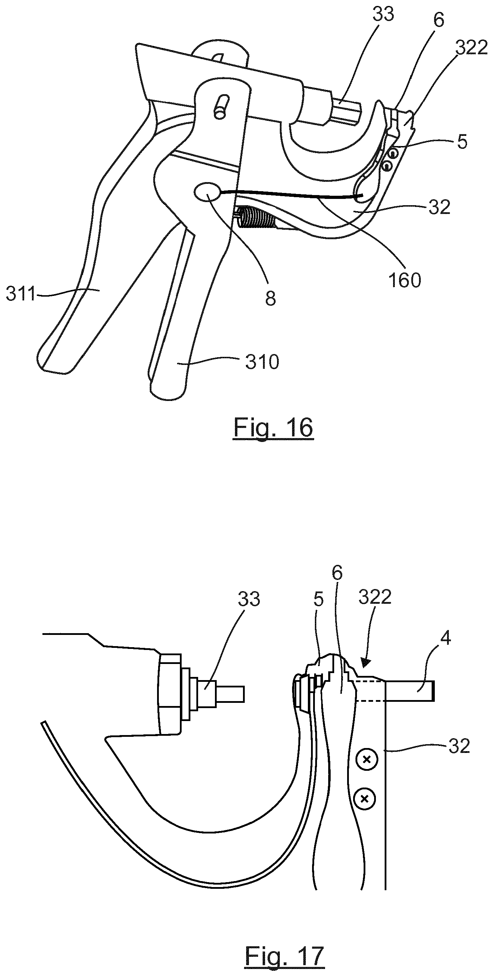

La mise en œuvre de moyens de maintien à commande déportée vient d'être décrite dans le cadre d'une pince pour la manipulation d'un dispositif d'identification d'un animal. De tels moyens de maintien à commande déportée peuvent toutefois également être mis en œuvre dans le cadre d'une pince pour la manipulation d'un dispositif de prélèvement d'un tissu d'un animal telle que celle illustrée aux figures 16 et 17.

Dans ce cas, l'une des mâchoires, en l'occurrence la mâchoire 32 est fixe alors que l'autre mâchoire 33 est mobile pour être approchée ou éloignée de la mâchoire 32.

La mâchoire 32 présente une ouverture latérale d'extrémité 322 dans laquelle peut-être insérée latéralement un tube de prélèvement 4 (récipient).

La mâchoire 33 est ici un piston qui permet de découper et de pousser un tissu d'un animal dans le tube de prélèvement 4.

Le tube de prélèvement 4 peut-être maintenu solidaire de la mâchoire 32 au moyen d'une paire de mors 5, 6 placés en regard l'un de l'autre de part et d'autre de l'ouverture 322.

Ces mors 5, 6 sont montés mobiles entre :
- une position de libération dans laquelle leurs extrémités libres sont éloignées l'une de l'autre pour libérer l'ouverture 322 et ainsi permettre l'introduction latérale d'un tube de prélèvement 4 dans celle-ci ou bien extraire un tube de prélèvement 4 de celle-ci ;
- une position de maintien dans laquelle leurs extrémités libres sont rapprochées l'une de l'autre pour refermer l'ouverture 322 et ainsi maintenir enserrer un tube de prélèvement 4 dans celle-ci.

Un bouton d'actionnement 8, déporté au niveau de l'extrémité des branches 310, 311 permet d'actionner à distance les mors 5, 6 pour les faire passer d'une à l'autre de leur position.

Ce bouton d'actionnement 8 est relié aux mors 5, 6 au moyen d'une transmission 160 de sorte que l'actionnement du bouton 8 permette de déplacer les mors 5, 6 de l'une à l'autre de leur position. Il pourra par exemple s'agir d'une transmission souple et/ou rigide. A titre d'exemple, il pourrait s'agir d'une transmission à tringle(s), à câble(s) ou autre.

Pour effectuer un prélèvement, un opérateur saisi d'une main la pince et, alors que les mâchoires 32, 33 sont éloignées, agit sur le bouton d'actionnement 8 pour placer les mors 5, 6 dans leur position de libération. Il peut alors positionner de l'autre main un tube de prélèvement 4 dans l'ouverture latérale 322 et l'y maintenir puis agir sur le bouton d'actionnement 8 de l'autre main pour placer les mors 5, 6 dans leur position de maintien. L'opérateur peut ensuite placer entre les mâchoires 32, 33 une partie d'un animal comme par exemple une oreille. Il actionne ensuite les branches 310, 311 de sorte à rapprocher l'une de l'autre les mâchoires 32, 33. La mâchoire 33 découpe alors un morceau de tissu de l'oreille et le pousse dans le tube de prélèvement 4. L'opérateur relâche ensuite la pince pour éloigner les mâchoires l'une de l'autre et dégager la pince de l'animal puis agit sur le bouton d'actionnement 8 pour placer les mors 5, 6 dans leur position de libération et ainsi récupérer le tube de prélèvement 4.

Dans une variante, le bouton d'actionnement 8 permettra à un opérateur de placer les mors 5, 6 dans leur position de maintien contre l'effet de moyens de rappel élastique. Dans ce cas, le fait de relâcher le bouton d'actionnement 8 permettra aux mors 5, 6 de retourner dans leur position de libération sous l'effet des moyens de rappel élastique. Ce fonctionnement pourrait être inversé.

Certains dispositifs de prélèvement permettent également la pose d'un dispositif d'identification.

## Revendications

1. Pince (3) pour la manipulation d'un dispositif d'identification d'un animal et/ou de prélèvement d'un tissu d'un animal, ladite pince (3) comprenant deux mâchoires (32, 33), une poignée (31) permettant de rapprocher lesdites mâchoires (32, 33), et des moyens de maintien (321) d'un élément dudit dispositif d'identification et/ou de prélèvement solidaire d'une desdites mâchoires (32, 33), lesdits moyens de maintien (321) étant mobiles entre :
- une position de libération dans laquelle ils ne maintiennent pas solidaire de ladite mâchoire (32, 33) ledit élément dudit dispositif d'identification et/ou de prélèvement, et
- une position de maintien dans laquelle ils maintiennent ledit élément dudit dispositif d'identification et/ou de prélèvement solidaire de ladite mâchoire (32, 33) par enserrement,
**caractérisée en ce que** ladite pince (3) comprend des moyens d'actionnement (73) pour déplacer lesdits moyens de maintien (321) d'une à l'autre de leur position, lesdits moyens d'actionnement (73) étant actionnables manuellement depuis ladite poignée (31) au moyen d'une main enserrant ladite poignée (31).

2. Pince selon la revendication 1, ledit dispositif d'identification comprenant un élément femelle (1) et un élément mâle (2) destinés à être placés de part et d'autre d'une oreille (O) d'un animal et à être rapproché pour coopérer entre eux et maintenir ledit dispositif d'identification solidaire de ladite oreille (O), lesdits éléments femelle (1) et mâle (2) étant chacun destiné à être supportés par une desdites mâchoires (32, 33), lesdits moyens de maintien (321) étant mobiles entre :
- une position de libération dans laquelle lesdits moyens de maintien sont éloignés de ladite mâchoire (32, 33) correspondante, et
- une position de maintien dans laquelle lesdits moyens de maintien (321) sont rapprochés de ladite mâchoire (32, 33) correspondante pour maintenir le cas échéant ledit élément mâle (2) ou ledit élément femelle (1) entre lesdits moyens de maintien (321) et ladite mâchoire (32, 33) correspondante.

3. Pince selon la revendication 1 ou 2 dans laquelle lesdits moyens de maintien (321) sont montés mobiles autour d'un premier axe de rotation (Z) entre leur position de maintien et leur position de libération.

4. Pince selon l'une quelconque des revendications 1 à 2 dans laquelle lesdits moyens de maintien (321) sont montés mobiles selon un axe de translation (B) entre leur position de maintien et leur position de libération.

5. Pince selon la revendication 3 ou 4 dans laquelle ladite poignée (31) comprend deux branches (310, 311) montées mobile en rotation selon un deuxième axe de rotation (35), ledit premier axe de rotation (Z) étant essentiellement parallèle audit deuxième axe de rotation (35) ou ledit axe de translation (B) étant essentiellement orthogonal audit deuxième axe de rotation (35).

6. Pince selon l'une quelconque des revendications 1 à 5 comprenant des moyens de rappel élastique (61) tendant à ramener lesdits moyens de maintien dans une de leur position, les moyens d'actionnement (73) permettant de les déplacer dans l'autre de leur position.

7. Pince selon la revendication 2 seule ou en combinaison avec l'une quelconque des revendications 3 à 6 dans laquelle lesdits moyens de maintien comprennent une bride (321) mobile entre :
- ladite position de libération dans laquelle ladite bride (321) et ladite mâchoire correspondante (32, 33) sont éloignées pour libérer ledit élément mâle (2) ou ledit élément femelle (1), et
- une position de maintien dans laquelle ladite bride (321) et ladite mâchoire correspondante (32, 33) sont rapprochées pour enserrer ledit élément mâle (2) ou ledit élément femelle (1) entre ladite bride (321) et ladite mâchoire correspondante (32, 33).

8. Pince selon l'une quelconque des 1 à 7 dans laquelle lesdits moyens d'actionnement comprennent au moins un levier (73) s'étendant à l'extrémité de ladite poignée (31) située du côté desdites mâchoires (32, 33) de manière à pouvoir être actionné par un doigt d'une main empoignant ladite poignée (31).

9. Pince selon les revendications 7 et 8 dans laquelle ledit au moins un levier (73) est solidaire d'un flanc (7) dont est solidaire ladite bride (321).

10. Pince selon la revendication 9 dans laquelle ledit levier (73) forme avec ledit flanc (7) et ladite bride (321) un ensemble monobloc.

11. Pince selon la revendication 3 et l'une quelconque des revendications 9 ou 10 dans laquelle ledit flanc (7) porte au moins deux lumières (74) s'étirant le long d'un même cercle (C) dont le centre (A) passe par le premier axe de rotation (Z) des moyens de maintien (321), lesdites lumière (74) logeant des axes (75) de solidarisation dudit flanc(7) au reste de ladite pince (3).

12. Pince selon la revendication 4 et l'une quelconque des revendications 9 ou 10 dans laquelle ledit flanc (7) porte au moins deux lumières oblongue (74') s'étirant le long dudit axe de translation (B) des moyens de maintien, lesdites lumières oblongues (74') logeant des axes (75) de solidarisation dudit flanc (7) au reste de ladite pince (3).

13. Pince selon la revendication 11 dans laquelle lesdites lumières (74) ou lesdites lumières oblongues (74') sont latéralement décalées dudit premier axe de rotation (Z) en direction de ladite poignée (31).

14. Pince selon la revendication 1, ledit dispositif de prélèvement comprenant un récipient apte à recevoir un prélèvement, lesdits moyens de maintien comprenant deux mors (5, 6) placés en regard l'un de l'autre montés mobiles entre :
- une position de libération dans laquelle lesdits mors sont éloignés pour libérer ledit récipient, et
- une position de maintien dans laquelle lesdits mors sont rapprochés pour maintenir ledit récipient solidaire de ladite mâchoire (32, 33) correspondante.

## Patentansprüche

1. Zange (3) zur Handhabung einer Vorrichtung zur Kennzeichnung eines Tieres und/oder zur Entnahme eines Gewebes eines Tieres, wobei die Zange (3) zwei Backen (32, 33), einen Griff (31), der es ermöglicht, die Backen (32, 33) einander anzunähern, und Haltemittel (321) zum Halten eines Elements der Kennzeichnungs- und/oder Entnahmevorrichtung, das mit einer der Backen (32, 33) verbunden ist, umfasst, wobei die Haltemittel (321) beweglich sind zwischen:
- einer Freigabeposition, in der sie das Element der Kennzeichnungs- und/oder Entnahmevorrichtung nicht mit der Backe (32, 33) verbunden halten, und
- einer Halteposition, in der sie das Element der Kennzeichnungs- und/oder Entnahmevorrichtung durch Einspannen mit der Backe (32, 33) verbunden halten,
**dadurch gekennzeichnet, dass** die Zange (3) Betätigungsmittel (73) umfasst, um die Haltemittel (321) von einer Position in die andere zu bewegen, wobei die Betätigungsmittel (73) von dem Griff (31) aus manuell mit einer den Handgriff (31) einspannenden Hand betätigbar sind.

2. Zange nach Anspruch 1, wobei die Kennzeichnungsvorrichtung ein weibliches Element (1) und ein männliches Element (2) umfasst, die dazu bestimmt sind, beidseits eines Ohres (O) eines Tieres angeordnet und einander angenähert zu werden, um untereinander zusammenzuwirken und die Kennzeichnungsvorrichtung mit dem Ohr (O) verbunden zu halten, wobei das weibliche (1) und das männliche (2) Element jeweils dazu bestimmt sind, von einer der Backen (32, 33) getragen zu werden, wobei die Haltemittel (321) beweglich sind zwischen:
- einer Freigabeposition, in der die Haltemittel von der entsprechenden Backe (32, 33) beabstandet sind, und
- einer Halteposition, in der die Haltemittel (321) zu der entsprechenden Backe (32, 33) geführt sind, um gegebenenfalls das männliche Element (2) oder das weibliche Element (1) zwischen den Haltemitteln (321) und der entsprechenden Backe (32, 33) zu halten.

3. Zange nach Anspruch 1 oder 2, wobei die Haltemittel (321) zwischen ihrer Halteposition und ihrer Freigabeposition um eine erste Drehachse (Z) beweglich gelagert sind.

4. Zange nach einem der Ansprüche 1 bis 2, wobei die Haltemittel (321) zwischen ihrer Halteposition und ihrer Freigabeposition entlang einer Translationsachse (B) beweglich gelagert sind.

5. Zange nach Anspruch 3 oder 4, wobei der Griff (31) zwei Schenkel (310, 311) umfasst, die entlang einer zweiten Drehachse (35) beweglich gelagert sind, wobei die erste Drehachse (Z) im Wesentlichen parallel zu der zweiten Drehachse (35) verläuft oder die Translationsachse (B) im Wesentlichen orthogonal zu der zweiten Drehachse (35) verläuft.

6. Zange nach einem der Ansprüche 1 bis 5, umfassend elastische Rückstellmittel (61), die bestrebt sind, die Haltemittel in eine ihrer Positionen zurückzuführen, wobei die Betätigungsmittel (73) es ermöglichen, sie in die andere ihrer Positionen zu bewegen.

7. Zange nach Anspruch 2 allein oder in Kombination mit einem der Ansprüche 3 bis 6, wobei die Haltemittel einen Bügel (321) umfassen, der beweglich ist zwischen:
- der Freigabeposition, in welcher der Bügel (321) und die entsprechende Backe (32, 33) voneinander beabstandet sind, um das männliche Element (2) oder das weibliche Element (1) freizugeben, und
- einer Halteposition, in welcher der Bügel (321) und die entsprechende Backe (32, 33) einander angenähert sind, um das männliche Element (2) oder das weibliche Element (1) zwischen dem Bügel (321) und der entsprechenden Backe (32, 33) einzuspannen.

8. Zange nach einem der Ansprüche 1 bis 7, wobei die Betätigungsmittel mindestens einen Hebel (73) umfassen, der sich an dem Ende des Griffs (31) erstreckt, das auf der Seite der Backen (32, 33) gelegen ist, so dass er durch einen Finger einer den Griff (31) erfassenden Hand betätigt werden kann.

9. Zange nach den Ansprüchen 7 und 8, wobei der mindestens eine Hebel (73) mit einer Flanke (7) verbunden ist, mit welcher der Bügel (321) verbunden ist.

10. Zange nach Anspruch 9, wobei der Hebel (73) mit der Flanke (7) und dem Bügel (321) eine einstückige Anordnung bildet.

11. Zange nach Anspruch 3 und einem der Ansprüche 9 oder 10, wobei die Flanke (7) mindestens zwei Schlitze (74) trägt, die sich entlang eines selben Kreises (C) erstrecken, dessen Mittelpunkt (A) durch die erste Drehachse (Z) der Haltemittel (321) verläuft, wobei die Schlitze (74) Stifte (75) zum Verbinden der Flanke (7) mit dem Rest der Zange (3) aufnehmen.

12. Zange nach Anspruch 4 und einem der Ansprüche 9 oder 10, wobei die Flanke (7) mindestens zwei längliche Schlitze (74') trägt, die sich entlang der Translationsachse (B) der Haltemittel erstrecken, wobei die länglichen Schlitze (74') Stifte (75) zum festen Verbinden der Flanke (7) mit dem Rest der Zange (3) aufnehmen.

13. Zange nach Anspruch 11, wobei die Schlitze (74) oder die länglichen Schlitze (74') seitlich von der ersten Drehachse (Z) in Richtung des Griffs (31) versetzt sind.

14. Zange nach Anspruch 1, wobei die Entnahmevorrichtung einen Behälter umfasst, der geeignet ist, eine Entnahme aufzunehmen, wobei die Haltemittel zwei Greifflächen (5, 6) umfassen, die einander gegenüber angeordnet sind und beweglich gelagert sind zwischen:
- einer Freigabeposition, in der die Greifflächen voneinander beabstandet sind, um den Behälter freizugeben, und
- einer Halteposition, in der die Greifflächen einander angenähert sind, um den Behälter mit der entsprechenden Backe (32, 33) verbunden zu halten.

## Claims

1. Clamp (3) for manipulating a device for identifying an animal and/or sampling tissue from an animal, said clamp (3) comprising two jaws (32, 33), a handle (31) making it possible to bring said jaws (32, 33) closer together, and means (321) for holding an element of said identification and/or sampling device secured to one of said jaws (32, 33), said holding means (321) being able to move between:
- a release position in which they do not hold said element of said identification and/or sampling device secured to said jaw (32, 33), and
- a holding position in which they hold said element of said identification and/or sampling device secured to said jaw (32, 33) by gripping,
**characterized in that** said clamp (3) comprises actuating means (73) for moving said holding means (321) from one of their positions to the other, said actuating means (73) being able to be actuated manually from said handle (31) by means of a hand gripping said handle (31).

2. Clamp according to Claim 1, said identification device comprising a female element (1) and a male element (2) that are intended to be placed on either side of an ear (O) of an animal and to be brought closer together to cooperate with each other and hold said identification device secured to said ear (O), said female (1) and male (2) elements each being intended to be supported by one of said jaws (32, 33), said holding means (321) being able to move between:
- a release position in which said holding means are spaced apart from said corresponding jaw (32, 33), and
- a holding position in which said holding means (321) are close to said corresponding jaw (32, 33) to hold, if appropriate, said male element (2) or said female element (1) between said holding means (321) and said corresponding jaw (32, 33).

3. Clamp according to Claim 1 or 2, wherein said holding means (321) are mounted so as to be able to move about a first axis of rotation (Z) between their holding position and their release position.

4. Clamp according to either one of Claims 1 and 2, wherein said holding means (321) are mounted so as to be able to move along an axis of translation (B) between their holding position and their release position.

5. Clamp according to Claim 3 or 4, wherein said handle (31) comprises two branches (310, 311) mounted so as to be able to move in rotation about a second axis of rotation (35), said first axis of rotation (Z) being essentially parallel to said second axis of rotation (35) or said axis of translation (B) being essentially orthogonal to said second axis of rotation (35).

6. Clamp according to any one of Claims 1 to 5, comprising elastic return means (61) that tend to return said holding means to one of their positions, the actuating means (73) making it possible to move them to the other of their positions.

7. Clamp according to Claim 2 alone or in combination with any one of Claims 3 to 6, wherein said holding means comprises a flange (321) that is able to move between:
- said release position in which said flange (321) and said corresponding jaw (32, 33) are spaced apart to release said male element (2) or said female element (1), and
- a holding position in which said flange (321) and said corresponding jaw (32, 33) are close together to grip said male element (2) or said female element (1) between said flange (321) and said corresponding jaw (32, 33).

8. Clamp according to any one of Claims 1 to 7, wherein said actuating means comprise at least one lever (73) extending at the end of said handle (31) that is situated on the side of said jaws (32, 33) so as to be able to be actuated by a finger of a hand grasping said handle (31).

9. Clamp according to Claims 7 and 8, wherein said at least one lever (73) is as one with a flank (7) with which said flange (321) is as one.

10. Clamp according to Claim 9, wherein said lever (73) forms a one-piece assembly with said flank (7) and said flange (321).

11. Clamp according to Claim 3 and either one of Claims 9 and 10, wherein said flank (7) bears at least two slots (74) extending along one and the same circle (C), the centre (A) of which passes through the first axis of rotation (Z) of the holding means (321), said slots (74) housing pins (75) for securing said flank (7) to the rest of said clamp (3).

12. Clamp according to Claim 4 and either one of Claims 9 and 10, wherein said flank (7) bears at least two oblong slots (74') extending along said axis of translation (B) of the holding means, said oblong slots (74') housing pins (75) for securing said flank (7) to the rest of said clamp (3).

13. Clamp according to Claim 11, wherein said slots (74) or said oblong slots (74') are laterally offset from said first axis of rotation (Z) in the direction of said handle (31).

14. Clamp according to Claim 1, said sampling device comprising a container that is able to receive a sample, said holding means comprising two jaw pieces (5, 6) placed facing one another and mounted so as to be able to move between:
- a release position in which said jaw pieces are spaced apart to release said container, and
- a holding position in which said jaw pieces are close together to hold said container secured to said corresponding jaw (32, 33).
